# Europäisches Patentamt

# European Patent Office

# Office européen des brevets

(19)

(11) Veröffentlichungsnummer: **0 008 116**
**B1**

(12) ## EUROPÄISCHE PATENTSCHRIFT

(45) Veröffentlichungstag der Patentschrift:
09.02.83

(51) Int. Cl.³: **C 07 D 303/44**, C 07 D 301/14

(21) Anmeldenummer: **79102861.6**

(22) Anmeldetag: **08.08.79**

(54) **Verfahren zur Herstellung von 7-Oxabicyclo(4.1.0)heptan-3,4-dicarbonsäure-diglycidylester.**

(30) Priorität: **16.08.78 DE 2835884**

(43) Veröffentlichungstag der Anmeldung:
**20.02.80 Patentblatt 80/4**

(45) Bekanntmachung des Hinweises auf die Patenterteilung:
**09.02.83 Patentblatt 83/6**

(84) Benannte Vertragsstaaten:
**CH DE FR GB IT NL**

(56) Entgegenhaltungen:
**DE-A-1 942 557**
**DE-A-2 602 776**
**DE-B-1 082 263**
**DE-B-1 083 797**
**FR-A-1 269 628**

(73) Patentinhaber: **BAYER AG, Zentralbereich Patente, Marken und Lizenzen, D-5090 Leverkusen 1, Bayerwerk (DE)**

(72) Erfinder: **Rauleder, Gebhard, Dr., Mozartstrasse 20, D-5657 Haan (DE)**
Erfinder: **Waldmann, Helmut, Dr., Carl-Rumpff-Strasse 59, D-5090 Leverkusen 1 (DE)**
Erfinder: **Bottenbruch, Ludwig, Dr., Woehlerstrasse 5, D-4150 Krefeld 1 (DE)**
Erfinder: **Traenckner, Hans-Joachim, Dr., Wedelstrasse 46, D-4150 Krefeld 1 (DE)**
Erfinder: **Seifert, Hermann, Dr., Ruwergasse 4, D-5000 Koeln 80 (DE)**
Erfinder: **Swodenk, Wolfgang, Dr., Auf dem Broich 5, D-5068 Odenthal (DE)**

BUNDESDRUCKEREI BERLIN

## Verfahren zur Herstellung von 7-Oxabicyclo(4.1.0)heptan-3,4-dicarbonsäure-diglycidylester

Die vorliegende Erfindung betrifft ein Verfahren zur Herstellung von 7-Oxabicyclo(4.1.0)heptan-3,4-dicarbonsäurediglycidylester aus Tetrahydrophthalsäurediallylester.

7-Oxabicyclo(4.1.0)heptan-3,4-dicarbonsäurediglycidylester kann als Ausgangsprodukt zur Herstellung von Epoxyharzen, von Polyäthern und Polyurethanen, zur Herstellung von Lacken und Kunststoffen, als Weichmacher, als Stabilisator und als organisches Zwischenprodukt Verwendung finden (s. z. B. DE-A-1 082 263, US-A-2 870 179 und Proc. Anniv. Conf. SPI (Soc. Plast Ind.) Reinf. Plast. Comp. Div. 25th, 1970, 3-B, S. 1—8). Ein technischer Einsatz dieses Produktes ist in größerem Maße bisher nicht erfolgt, da kein befriedigendes technisches Verfahren zu seiner Herstellung zur Verfügung gestanden hat.

Eine Möglichkeit, Olefine in Epoxide umzuwandeln besteht in der Anwendung der »Prileschajew-Reaktion« (vgl. N. Prileschajew, Ber.dtsch. chem. Ges. 42, 4811, (1909)). Bei dieser Reaktion handelt es sich um einen elektrophilen Angriff einer Percarbonsäure auf ein Olefin (vgl. K. D. Bingham, G. D. Meakins, G. H. Whitham, Chem. Commun. 1966, S. 445 und 446). Aus diesem Grunde nimmt die Reaktivität des Olefins mit fallender Nucleophilie der Doppelbindung ab. Deshalb erschweren elektronegative Substituenten die Epoxidation (vgl. S. N. Lewis in R. L. Augustin, »Oxidation« Vol. I, S. 227, Z. 9—13, Marcel Dekker, New York (1969)).

Elektronenziehende Gruppierungen wie z. B. Carboxyl, Carbonyl usw. vermindern die Reaktionsgeschwindigkeit enorm (vgl. H. Batzer und E. Nikles, Chimia, Vol. 16, Seite 62 (1962)). Insbesondere lassen sich daher Allylester nicht ohne weiteres mit Percarbonsäuren epoxidieren (vgl. DE-A-2 023 148). Infolge der geringeren Reaktionsfähigkeit ihrer Doppelbindung sind hohe Temperaturen und lange Reaktionszeiten erforderlich, was zur Bildung von unerwünschten Nebenprodukten, wie Dihydroxy- und Hydroxyacyloxy-Derivaten der Ausgangsprodukte, Anlaß gibt (vgl. S. N. Lewis in R. L. Augustin. »Oxidation« Vol. 1, S. 233, Z. 6—11, Marcel Dekker, New York (1969)).

Im Tetrahydrophthalsäurediallylester ist die Reaktivität der Ringdoppelbindung bezüglich elektrophiler Angriffe bedeutend größer als die der allylischen Doppelbindungen. Es ist deshalb nicht ohne weiteres möglich, 7-Oxabicyclo(4.1.0)heptan-3,4-dicarbonsäurediglycidylester durch Umsetzung von Tetrahydrophthalsäurediallylester mit einer Percarbonsäure herzustellen.

So wird in der US-A-2 783 250, Beispiel 2, die Umsetzung von Tetrahydrophthalsäurediallylester mit Peressigsäure in Chloroform beschrieben. Nach einer Reaktionsdauer von 10 Stunden bei 0 bis 10°C wurde nach dem Abtrennen der Essigsäure und des Lösungsmittels ein hochviskoser Rückstand erhalten, der als 7-Oxabicyclo(4.1.0)heptan-3,4-dicarbonsäurediallylester identifiziert wurde.

Über die Umsetzung von Tetrahydrophthalsäurediallylester mit einer Lösung von Peressigsäure in Essigsäure wird in J. Chem. Soc. 1952, S. 4631 berichtet. Nach einer Reaktionszeit von 20 Stunden bei 20—25°C wurde 7-Oxabicyclo(4.1.0)heptan-3,4-dicarbonsäurediallylester in einer Ausbeute von 65% erhalten.

In der JP-B-76/000 108 wird die Umsetzung von Tetrahydrophthalsäurediallylester mit Wasserstoffperoxid in Gegenwart von Ameisensäure und Katalysatoren beschrieben. Das Reaktionsprodukt ist auch hier 7-Oxabicyclo(4.1.0)heptan-3,4-dicarbonsäurediallylester.

In der US-A-2 794 030 wird die Umsetzung von Tetrahydrophthalsäurediallylester mit Peressigsäure in Aceton bei 25—30°C beschrieben (Beispiel 12). Nach einer Gesamtreaktionszeit von etwa 6 Stunden wurde 7-Oxabicyclo(4.1.0)heptan-3,4-dicarbonsäurediallylester in einer Reinheit von 95,5% erhalten.

In der US-A-2 870 170, Beispiel 1, wird die Umsetzung von Tetrahydrophthalsäurediallylester mit Peressigsäure in Chloroform beschrieben. Nach einer Reaktionszeit von 2 Tagen bei 0 bis 10°C wurde nach Abtrennen der Essigsäure und des Lösungsmittels ein Rückstand erhalten, der als 7-Oxabicyclo(4.1.0)-3,4-dicarbonsäure-allyl-glycidylester indentifiziert wurde. Die Ausbeute betrug 50% d. Th.

Aus der DE-B-1 083 797 ist ein Verfahren zur Herstellung von Epoxiden durch Umsetzung von Monoolefinen mit 7 bis 12 C-Atomen mit Peressigsäure bekannt, bei dem man das Reaktionsgemisch in Gegenwart eines Überschusses an Olefin destilliert, der ausreicht, um die entstandene Essigsäure als azeotropes Gemisch zu entfernen. Diese Destillation wird bei Temperaturen unter 100°C durchgeführt, und man kann in Gegenwart eines über dem Siedepunkt des Epoxids siedenden Treibmittels arbeiten. Das erfindungsgemäße Verfahren unterscheidet sich von diesem Verfahren wesentlich. So entstehen erfindungsgemäß höher als Essigsäure siedende Carbonsäuren, von denen nicht bekannt ist, ob sie mit Tetrahydrophthalsäurediallylester Azeotrope bilden können. Solche Azeotrope hätten in jedem Fall aber wesentlich höhere Siedepunkte als die gemäß der DE-B-1 083 797 auftretenden. Außerdem kann der erfindungsgemäß einzusetzende Allylester nicht im Überschuß eingesetzt werden, weil sonst die Bildung eines Triepoxids nicht möglich ist. Der erfindungsgemäß zuzufügende Zusatzstoff soll zwischen der entstehenden Carbonsäure und dem nächst höher siedenden Bestandteil sieden, nicht oberhalb des gebildeten Epoxids. Erfindungsgemäß kann die Destillation nicht auf Temperaturen unter 100°C begrenzt werden, in der DE-B-1 083 797 wird dagegen ausgesagt, daß bereits über 50°C die gebildete Carbonsäure Epoxide aufspalten kann, und erfindungsgemäß liegen Tris-Epoxide vor, die erhöhte Angriffsmöglichkeiten haben im Vergleich zu den Monoepoxiden, die gemäß der DE-B-1 083 797 erhalten werden. Schließlich geht aus dieser Druckschrift nicht hervor, daß das dort beschriebene Verfahren zur Epoxidation von drei epoxidierbare Gruppen enthaltenden Verbindungen geeignet ist. Aus allen diesen Gründen kann dieses bekannte Verfahren das erfindungsgemäße Verfahren nicht nahelegen.

Es wurde nun ein Verfahren zur Herstellung von 7-Oxabicyclo(4.1.0)heptan-3,4-dicarbonsäurediglycidylester gefunden, das dadurch gekennzeichnet ist, daß man Tetrahydrophthalsäurediallylester mit einer 3 bis 4 Kohlenstoffatome enthaltenden Percarbonsäure in Gegenwart von organischen Lösungsmitteln bei einer Temperatur von 0 bis 100°C umsetzt und anschließend das erhaltene Reaktionsgemisch durch Destillation aufarbeitet, wobei man die aus der Percarbonsäure entstandene Carbonsäure abtrennt, indem man dem Reaktionsgemisch einen Zusatzstoff zufügt, der zwischen der aus Percarbonsäure entstehenden Carbonsäure und dem nächst höher siedenden Bestandteil des Reaktionsgemisches siedet und die Carbonsäure zusammen mit dem Lösungsmittel abdestilliert.

Nach dem erfindungsgemäßen Verfahren einsetzbarer Tetrahydrophthalsäure-diallylester kann in an sich bekannter Weise erhalten werden, beispielsweise durch Veresterung von Tetrahydrophthalsäureanhydrid mit Allylalkohol.

Als organische Lösungsmittel können im erfindungsgemäßen Verfahren die verschiedensten unsubstituierten und substituierten Kohlenwasserstoffe verwendet werden, die unter Reaktionsbedingungen flüssig sind und unerwünschte Nebenreaktionen nicht oder nur in ganz untergeordnetem Maße eingehen. Als Kohlenwasserstoffe können z. B. verwendet werden aliphatische und cycloaliphatische Kohlenwasserstoffe wie Hexan, Heptan, Octan, 2-Äthyl-hexan, Decan, Dodecan, Cyclohexan, Methylcyclopentan und Petroläther, weiterhin aromatische Kohlenwasserstoffe wie Benzol, Nitrobenzol, Toluol, Äthylbenzol, Cumol, Diisopropylbenzol, Xylol und Chlorbenzol, weiterhin sauerstoffhaltige Kohlenwasserstoffe wie Diäthyläther, Diisopropyläther, Dibutyläther, Tetrahydrofuran, Dioxan, Essigsäureäthylester, Essigsäuremethylester, Essigsäurepropylester, Essigsäurebutylester, Propionsäuremethylester, Propionsäureäthylester, Propionsäurepropylester, Buttersäuremethylester, Buttersäureäthylester, Buttersäurepropylester, Buttersäurebutylester, Benzoesäuremethylester und Benzoesäureäthylester, weiterhin chlorierte Kohlenwasserstoffe wie Methylenchlorid, Chloroform, Tetrachlorkohlenstoff, 1-Chloräthan, 1,2-Dichloräthan, 1,1-Dichloräthan, 1,1,2,2-Tetrachloräthan, 1-Chlorpropan, 2-Chlorpropan, 1,2-Dichlorpropan, 1,3-Dichlorpropan, 2,3-Dichlorpropan, 1,2,3-Trichlorpropan, 1,1,2,3-Tetrachlorpropan, Butylchlorid, 1,2-Dichlorbutan, 1,4-Dichlorbutan, 2,3-Dichlorbutan, 1,3-Dichlorbutan, 1,2,3,4-Tetrachlorbutan, tert.-Butylchlorid, Amylchlorid, 1,2-Dichlorpentan, 1,5-Dichlorpentan, 1,2,3,4-Tetrachlorpentan, Cyclopentylchlorid, 1,2-Dichlorcyclopentylchlorid, Hexylchlorid, 1,2-Dichlorhexan, 1,6-Dichlorhexan, 1,2,3,4-Tetrachlorhexan, 1,2,5,6-Tetrachlorhexan, Cyclohexylchlorid, Chlorbenzol, Heptylchlorid, 1,2-Dichlorheptan, 1,2,3,4-Tetrachlorheptan, Cycloheptylchlorid, Octylchlorid, 1,2-Dichloroctan, 1,2,3,4-Tetrachloroctan und Cyclooctylchlorid.

Bevorzugte Lösungsmittel sind von den chlorierten Kohlenwasserstoffen Methylenchlorid, Chloroform, Tetrachlorkohlenstoff und 1,2-Dichlorpropan, von den aromatischen Kohlenwasserstoffen Benzol, Nitrobenzol, Toluol und Chlorbenzol, von den aliphatischen und cycloaliphatischen Kohlenwasserstoffen 2-Äthyl-hexan, Cyclohexan und Methylcyclopentan und von den sauerstoffhaltigen Kohlenwasserstoffen Tetrahydrofuran, Propionsäureäthylester und Benzoesäureäthylester.

Besonders bevorzugte Lösungsmittel sind von den chlorierten Kohlenwasserstoffen 1,2-Dichlorpropan und Tetrachlorkohlenstoff, von den aromatischen Kohlenwasserstoffen Benzol und Chlorbenzol, von den aliphatischen und cycloaliphatischen Kohlenwasserstoffen Cyclohexan und von den sauerstoffhaltigen Kohlenwasserstoffen Proipionsäureäthylester und Benzoesäureäthylester.

Verwendet werden können auch Lösungsmittelgemische der verschiedenen oben angegebenen organischen Lösungsmittel.

Erfindungsgemäß verwendbare Percarbonsäuren sind Perpropionsäure, Perbuttersäure und Perisobuttersäure. Bevorzugt verwendet werden Perpropionsäure und Perisobuttersäure. Besonders bevorzugt ist Perpropionsäure. Diese Percarbonsäuren, gelöst in einem der genannten organischen Lösungsmittel, können z. B. nach dem in der DE-A-2 262 970 beschriebenen Verfahren hergestellt werden, bei dem wäßriges Wasserstoffperoxid mit der entsprechenden Carbonsäure in Gegenwart von Schwefelsäure umgesetzt und anschließend die entstandene Percarbonsäure mit einem organischen Lösungsmittel aus dem Reaktionsgemisch extrahiert wird. Gegebenenfalls kann die so erhaltene Percarbonsäurelösung in dem organischen Lösungsmittel noch weiter gereinigt werden, insbesondere um den Gehalt an Wasser, Wasserstoffperoxid und Schwefelsäure zu erniedrigen.

Im allgemeinen verwendet man die Percarbonsäuren in Form einer Lösung in einem organischen Lösungsmittel. Derartige Percarbonsäurelösungen können beispielsweise 10 bis 30 Gew.-% der jeweiligen Percarbonsäure, bezogen auf die Lösung, enthalten. Die Allylester können als solche oder ebenfalls gelöst in einem oder mehreren der vorstehend genannten Lösungsmittel eingesetzt werden, wobei beliebig konzentrierte Lösungen der Allylester zum Einsatz gelangen können. Vorzugsweise werden die Allylester als solche eingesetzt und organische Lösungsmittel nur in Form der Percarbonsäurelösung zugegeben.

Das Molverhältnis von eingesetzter Percarbonsäure zu eingesetztem Tetrahydrophthalsäurediallylester kann in weiten Grenzen schwanken. Beispielsweise kann dieses Molverhältnis 1,5 : 1 bis 10 : 1 betragen. Bevorzugt wird ein Molverhältnis von 1,8 : 1 bis 7 : 1 angewendet. Ganz besonders vorteilhaft ist es, ein Molverhältnis von 2 bis 5 Mol Persäure je Mol Tetrahydrophthalsäurediallylester anzuwenden.

Der Wassergehalt der verwendeten Percarbonsäure soll im allgemeinen möglichst niedrig sein. Wassermengen bis 2 Gew.-% in der Percarbonsäurelösung sind im allgemeinen nicht störend. Geeignet ist beispielsweise eine Percarbonsäurelösung mit einem Wassergehalt von bis zu 1 Gew.-%. Vorzugsweise verwendet man eine Percarbonsäurelösung, die weniger als 0,3 Gew.-% Wasser enthält. Besonders bevorzugt ist ein Wassergehalt von weniger als 0,1 Gew.-%.

Der Wasserstoffperoxidgehalt der verwendeten Percarbonsäure in einem der obengenannten organischen Lösungsmittel soll im allgemeinen möglichst niedrig sein. Er kann beispielsweise bis zu 1 Gew.-%, bezogen auf die Percarbonsäurelösung, betragen. Vorteilhaft arbeitet man bei einem Gehalt von weniger als 0,5 Gew.-%. Besonders vorteilhaft ist es, die Umsetzung mit einer Percarbonsäurelösung durchzuführen, die einen Wasserstoffperoxidgehalt unterhalb 0,2 Gew.-% aufweist.

Der Mineralsäuregehalt der verwendeten Percarbonsäure soll möglichst niedrig sein. Vorteilhaft ist es, die Reaktion mit einer Percarbonsäurelösung durchzuführen, die einen Mineralsäuregehalt unterhalb 0,005 Gew.-% besitzt. Besonders vorteilhaft ist ein Mineralsäuregehalt von weniger als 0,001 Gew.-%.

Das erfindungsgemäße Verfahren wird in dem Temperaturbereich von 0−100°C durchgeführt. Bevorzugt arbeitet man bei 30−80°C, besonders bevorzugt bei 40−60°C. In Sonderfällen können die angegebenen Temperaturen auch unter- oder überschritten werden.

Neben der Arbeitsweise unter isothermen Bedingungen, d. h. Einhaltung einer einheitlichen Temperatur im gesamten Reaktionsgemisch, kann man das erfindungsgemäße Verfahren auch unter Ausbildung eines sogenannten Temperaturgradienten durchführen, der im allgemeinen mit fortschreitender Reaktion zunimmt. Man kann aber auch die Reaktion so führen, daß sich mit dem Fortschreiten der Reaktion ein Gradient fallender Temperatur ausbildet.

Das erfindungsgemäße Verfahren kann bei den verschiedensten Drücken durchgeführt werden. Im allgemeinen arbeitet man bei Normaldruck. Das Verfahren kann jedoch auch bei Unter- oder Überdruck durchgeführt werden.

Die Durchführung des erfindungsgemäßen Verfahrens kann diskontinuierlich oder kontinuierlich in den für Umsetzungen dieser Art üblichen Vorrichtungen, wie Rührwerkskessel, Siedereaktoren, Röhrenreaktoren, Schlaufenreaktoren oder Schleifenreaktoren, erfolgen.

Als Werkstoffe für die Reaktionsapparate zur Durchführung des erfindungsgemäßen Verfahrens können beispielsweise Glas, Edelstähle oder emailliertes Material verwendet werden.

Schwermetallionen im Reaktionsgemisch katalysieren die Zersetzung der Percarbonsäure. Man setzt deshalb der Percarbonsäurelösung im allgemeinen Substanzen zu, die Schwermetallionen durch Komplexbildung inaktivieren können. Bekannte Substanzen solcher Art sind beispielsweise Gluconsäure, Äthylendiamintetraessigsäure, Natriumsilicat, Natriumpyrophosphat, Natriumhexametaphosphat, Dinatriumdimethylpyrophosphat oder $Na_2$ (2-Äthyl-hexyl)$_5$($P_3O_{10}$)$_2$ (vgl. DE-B-1 056 596, Spalte 4, Zeile 60 ff.).

Die Reaktionswärme kann durch innen- oder außenliegende Kühler abgeführt werden. Zur Ableitung der Reaktionswärme kann die Umsetzung auch unter Rückfluß (z. B. in Siedereaktoren) durchgeführt werden.

Der Allylester und die Percarbonsäure können auf beliebige Weise zusammengebracht werden. Beispielsweise kann man beide Komponenten gleichzeitig oder nacheinander in beliebiger Reihenfolge in das Reaktionsgefäß einbringen. Bei diskontinuierlicher Arbeitsweise wird vorzugsweise der Allylester vorgelegt und dann die Percarbonsäurelösung zugegeben. Die Reaktionstemperatur kann dabei vor oder nach der Zugabe der Percarbonsäure eingestellt werden. Man kann auch die beiden Komponenten bei Raumtemperatur gleichzeitig in das Reaktionsgefäß eingeben und dann die Reaktionstemperatur einstellen. Bei kontinuierlicher Arbeitsweise kann man die beiden Komponenten gemeinsam oder getrennt dem Reaktor zuführen. Bei Verwendung mehrerer Reaktoren, die beispielsweise als Kaskade hintereinander geschaltet sein können, kann es vorteilhaft sein, den Allylester nur in den ersten Reaktor einzubringen. Man kann die Allylesterzugabe jedoch auch auf mehrere Reaktoren verteilen.

Es kann von Vorteil sein, den Tetrahydrophthalsäurediallylester durch chargenweise Zugabe von Percarbonsäure zu epoxidieren. Dabei kann es von Vorteil sein, partiell epoxidierten Tetrahydrophthalsäurediallylester aus dem Reaktionsgemisch abzutrennen und einer erneuten Umsetzung mit Percarbonsäure zu unterwerfen.

Bei der chargenweise Zugabe der Percarbonsäure kann es auch von Vorteil sein, nach Zugabe einzelner Chargen die aus der Percarbonsäure entstandene Carbonsäure aus dem Reaktionsgemisch zu entfernen, z. B. durch Extraktion mit Wasser oder durch Destillation.

Die nach Durchführung des erfindungsgemäßen Verfahrens erhaltenen Reaktionsgemische enthalten im allgemeinen das verwendete organische Lösungsmittel, die aus der Percarbonsäure entstehende Carbonsäure und 7-Oxabicyclo(4.1.0)-heptan-3,4-dicarbonsäurediglycidylester, wobei gegebenenfalls partiell epoxidierter und monoepoxidierter Tetrahydrophthalsäurediallylester sowie gegebenenfalls geringe Mengen von hochsiedenden Nebenprodukten im Reaktionsgemisch

4

vorhanden sein können.

Die Aufarbeitung des erhaltenen Reaktionsgemisches erfolgt durch Destillation. Dabei kann man so verfahren, daß man die einzelnen Komponenten in der Reihenfolge ihrer Siedepunkte abdestilliert. Dabei ist es von Vorteil, die Destillation im Vakuum und unter Verwendung von Verdampfern, die kurze Verweilzeiten und eine Verdampfung mit geringer thermischer Belastung des Reaktionsgemisches ermöglichen, durchzuführen. Erfindungsgemäß wird vor der destillativen Aufarbeitung dem Reaktionsgemisch ein geeigneter Zusatzstoff zugefügt, der zwischen der Carbonsäure und dem nächst höher siedenden Bestandteil des Reaktionsgemisches siedet. Dadurch erreicht man eine vollständige Abtrennung der Carbonsäure bei relativ niedrigen Sumpftemperaturen, was hinsichtlich der Minimierung der Bildung von Nebenprodukten aus der Carbonsäure und den epoxidierten Bestandteilen des Reaktionsgemisches von Vorteil ist. Erfindungsgemäß wird dann die Carbonsäure zusammen mit dem Lösungsmittel abdestilliert.

Es kann vorteilhaft sein, dem Reaktionsgemisch vor oder während der destillativen Aufarbeitung Stabilisatoren zuzusetzen, welche die Bildung von Hochsiedern und Polymerisaten verhindern.

Das erfindungsgemäße Verfahren hat eine Reihe von Vorteilen. Mit diesem Verfahren ist es erstmals möglich, nach der sogenannten Prileschajew-Reaktion in technischem Maßstab 7-Oxabicyclo(4.1.0)heptan-3,4-dicarbonsäurediglycidylester herzustellen.

Das folgende Beispiel erläutert die Erfindung. Sämtliche Prozentangaben sind, soweit nichts anderes gesagt wird, Gewichtsprozente.

## Beispiel

In einem 1-l-Dreihals-Doppelwand-Glaskolben mit Magnetrührer, Innenthermometer, Tropftrichter und Rückflußkühler werden 500 g Diallyltetrahydrophthalat (2 Mol) vorgelegt und auf 50°C thermostatisiert. Danach tropfte man 2142 g einer 20,7%igen Lösung von Perpropionsäure in Benzol (5 Mol) mit einem Wassergehalt von unter 0,1%, einem Wasserstoffperoxidgehalt von unter 0,2% und einem Mineralsäuregehalt von unter 0,001 Gew.-% so schnell zu, daß die Temperatur bei 50°C gehalten werden konnte. Nach Zutropfende wurde weiter bei dieser Temperatur gerührt. In zeitlichen Abständen wurden dem Reaktionsgemisch Proben entnommen und der zum jeweiligen Zeitpunkt noch vorhandene Gehalt an Perpropionsäure durch Titration, die Bildung des Epoxids bzw. die Abnahme des Olefins gaschromatographisch durch Zuwiegen eines internen Standards bestimmt.

Nach einer Reaktionszeit von 10 Stunden betrug der Persäureumsatz 96%, die Epoxidausbeute 90,1% d. Th., bezogen auf umgesetzte Perpropionsäure.

Zur Aufarbeitung wurde das Reaktionsgemisch mit 800 ml Benzoesäureäthylester vermischt und in einer Füllkörperkolonne mit Fallstromverdampfer fraktioniert.

Bei einem Vakuum von 200 mbar wurde Benzol zusammen mit Propionsäure als Kopfprodukt erhalten; es wurde in einer weiteren Kolonne in die Komponenten aufgetrennt.

Aus dem Sumpfprodukt wurde zunächst bei 20 mbar Benzoesäureäthylester abdestilliert, dann wurde das verbleibende Gemisch, das folgende Zusammensetzung besaß:

7-Oxabicyclo(4.1.0)heptan-3,4-dicarbonsäure-
| | |
|---|---|
| diallylester | 12% |
| allylglycidylester | 44% |
| diglycidylester | 36% |

bei einem Vakuum von 0,4 Torr fraktioniert. Monoepoxidierter und bisepoxidierter Tetrahydrophthalsäurediallylester wurden zur erneuten Umsetzung in den Reaktor zurückgeführt.

7-Oxabicyclo(4.1.0)heptan-3,4-dicarbonsäure-diglycidylester wurde in einer Reinheit von 99% erhalten.

Kp = 184 − 186°C bei 0,45 Torr.

Die Epoxidtitration ergab einen Epoxidsauerstoffgehalt von 16,02% (theor. 16,11)

Die Elementaranalyse ergab folgende Werte:

| | berechnet | gefunden |
|---|---|---|
| C: | 56,4% | 56,6% |
| H: | 6,1% | 6,2% |

## Patentansprüche

1. Verfahren zur Herstellung von 7-Oxabicyclo(4.1.0)heptan-3,4-dicarbonsäure-diglycidylester, dadurch gekennzeichnet, daß man Tetrahydrophthalsäurediallylester mit einer 3 bis 4 Kohlenstoffatome enthaltenden Percarbonsäure in Gegenwart von organischen Lösungsmitteln bei einer Temperatur von 0 bis 100°C umsetzt und anschließend das erhaltene Reaktionsgemisch durch Destillation aufarbeitet, wobei man die aus der Percarbonsäure entstandene Carbonsäure abtrennt, indem man dem Reaktionsgemisch einen Zusatzstoff zufügt, der zwischen der aus der Percarbonsäure entstehenden Carbonsäure und dem nächst höher siedenden Bestandteil des Reaktionsgemisches siedet und die Carbonsäure zusammen mit dem Lösungsmittel abdestilliert.

2. Verfahren nach Anspruch 1, dadurch gekennzeichnet, daß man Perpropionsäure einsetzt.

3. Verfahren nach Anspruch 1 und 2, dadurch gekennzeichnet, daß man die Umsetzung in Gegenwart von 1,2-Dichlorpropan, Tetrachlorkohlenstoff, Benzol, Chlorbenzol, Cyclohexan und/oder Propionsäureäthylester durchführt.

4. Verfahren nach Anspruch 1 bis 3, dadurch gekennzeichnet, daß man Percarbonsäure in Form einer Lösung in einem organischen Lösungsmittel verwendet, wobei die Lösung weniger als 2 Gew.-% Wasser, weniger als 1 Gew.-% Wasserstoffperoxid und weniger als 0,005 Gew.-% Mineralsäure enthält.

5. Verfahren nach Anspruch 1 bis 4, dadurch gekennzeichnet, daß man 1,5 bis 10 Mol Percarbonsäure pro Mol Tetrahydrophthalsäurediallylester anwendet.

6. Verfahren nach Anspruch 1 bis 5, dadurch gekennzeichnet, daß man dem Reaktionsgemisch vor oder während der destillativen Aufarbeitung Stabilisatoren zusetzt, welche die Bildung von Hochsiedern und Polymerisaten verhindern.

## Claims

1. Process for the preparation of 7-oxabicyclo(4.1.0)heptane-3,4-dicarboxylic acid diglycidyl ester, characterised in that tetrahydrophthalic acid diallyl ester is reacted with a percarboxylic acid containing 3 to 4 carbon atoms in the presence of organic solvents at a temperature of 0 to 100°C and the reaction mixture obtained is then worked up by distillation, the carboxylic acid formed from the percarboxylic acid being separated off by adding to the reaction mixture an additive which has a boiling point between that of the carboxylic acid forming from the percarboxylic acid and that of the constituent of the reaction mixture with the next highest boiling point and the carboxylic acid is distilled off together with the solvent.

2. Process according to claim 1, characterised in that perpropionic acid is employed.

3. Process according to claim 1 and 2, characterised in that the reaction is carried out in the presence of 1,2-dichloropropane, carbon tetrachloride, benzene, chlorobenzene, cyclohexane and/or ehtyl propionate.

4. Process according to claim 1 to 3, characterised in that the percarboxylic acid is used in the form of a solution in an organic solvent, the solution containing less than 2% by weight of water, less than 1% by weight of hydrogen peroxide and less than 0.005% by weight of mineral acid.

5. Process according to claim 1 to 4, characterised in that 1.5 to 10 mols of percarboxylic acid are used per mol of tetrahydrophthalic acid diallyl ester.

6. Process according to claim 1 to 5, characterised in that stabilisers which prevent the formation of high-boiling constituents and polymers are added to the reaction mixture before or during the working up by distillation.

## Revendications

1. Procédé de production d'ester de diglycidyle d'acide 7-oxabicyclo(4.1.0)heptane-3,4-dicarboxylique, caractérisé en ce qu'on fait réagir à une température de 0 à 100°C l'ester diallylique de l'acide tétrahydrophthalique avec un acide percarboxylique contenant 3 à 4 atomes de carbone, en présence de solvants organiques, puis on traite par distillation le mélange réactionnel obtenu, en séparant l'acide carboxylique produit à partir de l'acide percarboxylique, par addition au mélange réactionnel d'un additif qui bout entre l'acide carboxylique produit à partir de l'acid percarboxylique et le composant du mélange réactionnel à point d'ébullition immédiatement supérieur et on chasse l'acide carboxylique par distillation conjointement avec le solvant.

2. Procédé suivant la revendication 1, caractérisé en ce qu'on utilise l'acide perpropionique.

3. Procédé suivant les revendications 1 et 2, caractérisé en ce qu'on conduit la réaction en présence de 1,2-dichloropropane, de tétrachlorure de carbone, de benzène, de chlorobenzène, de cyclohexane et/ou de propionate d'éthyle.

4. Procédé suivant les revendications 1 à 3, caractérisé en ce qu'on utilise l'acide percarboxylique sous la forme d'une solution dans un solvant organique, cette solution contenant moins de 2% en poids d'eau, moins de 1% en poids de peroxyde d'hydrogène et moins de 0,005% en poids d'un acide minéral.

5. Procédé suivant les revendications 1 à 4, caractérisé en ce qu'on utilise 1,5 à 10 moles d'acide percarboxylique par mole d'ester diallylique d'acide tétrahydrophthalique.

6. Procédé suivant les revendications 1 à 5, caractérisé en ce qu'on ajoute au mélange réactionnel avant ou pendant le traitement par distillation, des agents stabilisants qui empêchent la formation de composés de haut point d'ébullition et de produits de polymérisation.